(19) Europäisches Patentamt
European Patent Office
Office européen des brevets

(11) EP 3 851 132 A1

(12) EUROPEAN PATENT APPLICATION
published in accordance with Art. 153(4) EPC

(43) Date of publication:
**21.07.2021 Bulletin 2021/29**

(21) Application number: **19859347.7**

(22) Date of filing: **13.09.2019**

(51) Int Cl.:
***A61L 27/44*** (2006.01) ***A61L 27/16*** (2006.01)
***A61L 27/18*** (2006.01) ***A61L 27/50*** (2006.01)
***A61L 27/56*** (2006.01) ***A61L 29/04*** (2006.01)
***A61L 29/06*** (2006.01) ***A61L 29/08*** (2006.01)
***A61L 29/12*** (2006.01) ***A61L 29/14*** (2006.01)
***A61L 31/04*** (2006.01) ***A61L 31/06*** (2006.01)
***A61L 31/10*** (2006.01) ***A61L 31/12*** (2006.01)
***A61L 31/14*** (2006.01)

(86) International application number:
**PCT/JP2019/036226**

(87) International publication number:
**WO 2020/054867 (19.03.2020 Gazette 2020/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **14.09.2018 JP 2018172258**

(71) Applicant: **Kyocera Corporation**
**Kyoto-shi, Kyoto 612-8501 (JP)**

(72) Inventors:
- **YAMANE, Shihori**
  **Kyoto-shi, Kyoto 612-8501 (JP)**
- **KYOMOTO, Masayuki**
  **Kyoto-shi, Kyoto 612-8501 (JP)**

(74) Representative: **Viering, Jentschura & Partner mbB**
**Patent- und Rechtsanwälte**
**Am Brauhaus 8**
**01099 Dresden (DE)**

# (54) MEDICAL MEMBER AND METHOD FOR MANUFACTURING SAME

(57) A polymer film having, covalently bonded thereto, a compound having a phosphorylcholine group is formed in a region which is difficult to be directly irradiated with light or a region which is not directly irradiated with light. A medical member is provided with a base material having light permeability and a polymer film arranged on at least a part of an inner surface of the base material and containing a polymeric chain formed as the result of the polymerization of a compound having a phosphorylcholine group, wherein the base material includes a first region which is at least a part of the inner surface and which light having an intensity sufficient to form the polymer film can reach through the base material when lights having specified intensities are emitted from the outer surface side of the base material.

*FIG.3A*

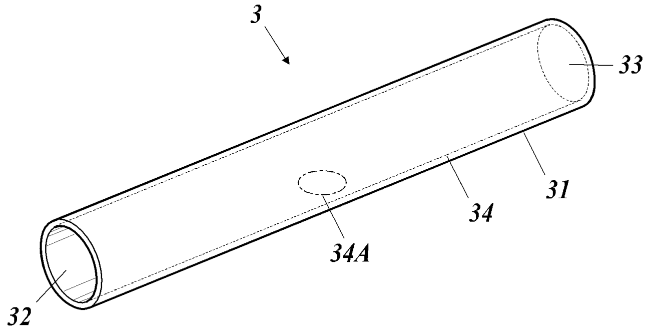


EP 3 851 132 A1

## Description

### Technical Field

The present disclosure relates to a medical member and a method for manufacturing the medical member.

### Background Art

A medical member such as an artificial joint and an artificial heart blood pump is known as a member used in a living body. Such a medical member has a base material made of resin, metal, or the like, and is further subjected to various surface treatments in order to have properties appropriate for its use. One of the surface treatments for a medical member widely performed in recent years is formation of a polymer layer with a polymer material having a phosphorylcholine group.

Therefore, one of the purposes is to form a polymer layer with a polymer material having a phosphorylcholine group accurately or efficiently.

### Summary of Invention

### Solution to Problem

A medical member according to the presently disclosed embodiment includes:

a base material that is permeable to light; and
a polymer layer that has a phosphorylcholine group, the polymer layer located at least at a part of an inner surface of the base material,
wherein the part of the inner surface includes a first area that is irradiated with light of an intensity capable of forming the polymer layer in response to being irradiated with light of a predetermined intensity from an outer surface side of the base material.

According to the presently disclosed embodiment, the method for manufacturing a medical member by the "grafting from" method (details will be described later) includes:

bringing an inner surface of a base material that is permeable to light into contact with a solution of a compound having a phosphorylcholine group,
irradiating the base material with light from an outer surface of the base material, and
forming a polymer layer having a phosphorylcholine group on at least at a part of the inner surface of the base material.

According to the presently disclosed embodiment, the method for manufacturing a medical member by the "grafting to" method (details will be described later) includes:

adsorbing a compound that has a photoreaction initiation group and a phosphorylcholine group on a surface of a base material that is permeable to light by bringing the surface of the base material into contact with a solution of the compound;
irradiating the base material with light having a light intensity that is enough to excite the photoreaction initiation group of the adsorbed compound; and
forming a polymer layer by covalently bonding the compound at least at a part of the surface of the base material, wherein the polymer layer is formed on a back side of the surface of the base material that is directly irradiated with the light.

### Brief Description of Drawings

FIG. 1A is a perspective view showing a medical member according to an embodiment of the present disclosure.
FIG. 1B is a cross-sectional view showing the medical member according to an embodiment of the present disclosure.
FIG. 2A is a perspective view showing a medical member according to an embodiment of the present disclosure.
FIG. 2B is a cross-sectional view showing the medical member according to the embodiment of the present disclosure.
FIG. 3A is a perspective view showing a medical member according to an embodiment of the present disclosure.

FIG. 3B is a perspective view showing a medical member according to an embodiment of the present disclosure.
FIG. 4 is a diagram showing a correlation between a thickness of a base material and an ultraviolet permeability.
Fig. 5 is a diagram showing a correlation between a density of a base material and an ultraviolet permeability.
FIG. 6 is a diagram showing results of Fourier transform infrared spectroscopic measurement of an exposed surface and a back exposed surface subjected to an MPC polymer treatment.
FIG. 7A is a TEM image of a cross section of an exposed surface of a base material subjected to the MPC polymer treatment.
FIG. 7B is a TEM image of a cross section of a back exposed surface of a base material subjected to the MPC polymer treatment.
FIG. 8 is a diagram showing a layer thickness of a formed MPC polymer layer as an ultraviolet irradiation intensity and an ultraviolet irradiation time are changed.
FIG. 9 is a diagram showing a layer thickness of a formed MPC polymer layer as an MPC concentration is changed.
FIG. 10 is a diagram showing a layer thickness of an MPC polymer layer formed by a "grafting to" method as an MPC concentration is changed.

Description of Embodiments

Hereinafter, embodiments of a medical member according to the present disclosure will be described in detail with reference to the drawings.

<Overview of Medical Member>

The medical member according to the present disclosure includes a base material that is permeable to light and a polymer layer that is located at least at a part of the inner surface of the base material. The polymer layer includes a polymer chain of a polymerized compound having a phosphorylcholine group. A first area of the base material is at least at the part of the inner surface, and is an area that is irradiated with, when irradiated with light of a predetermined intensity from outside of the base material, permeated light having an intensity capable of forming the polymer layer.
FIG. 1A to FIG. 3B are examples of medical members according to the presently disclosed embodiment.
FIG. 1A and FIG. 1B respectively show a perspective view and a cross-sectional view of a centrifugal blood pump 1 having a casing 11 that is a hollow light-permeating base material with openings (inlet port 12 and outlet port 13), an impeller 14 inside the casing 11, and the like, and a polymer layer (not shown) is provided at least on the inner surface of the casing 11 and on the surface of the impeller 14.
FIG. 2A and FIG. 2B respectively show a perspective view of a skull plate 2 and an enlarged cross sectional view of the skull plate 2 taken along a line $\alpha$-$\alpha$ in FIG. 2A. The skull plate 2 has polymer layers 24 on an outer surface(s) 22 of a base material 21 that is a light-permeating member and on an inner surface(s) 23A of the through hole(s) 23 that penetrates the base material 21.
FIG. 3A shows a medical member 3 having a base material 31 that is a hollow (cylindrical) light-permeating member having openings 32 and 33 at both ends and that has a polymer layer (not shown) formed on the entire inner surface 34 of the base material 31.
FIG. 3B shows a medical member 4 having a base material 41 that is a hollow square column having openings 42 and 43 at both ends and that has a polymer layer (not shown) formed on the entire inner surface 44 of the base material 41.
The medical member of the present disclosure includes, for example, a hollow medical member such as an artificial blood vessel, a hollow thread, an artificial lung, an artificial heart pump, a catheter, a stent, a dialyzer, a nerve induction tube, a shunt tube, and a spinal cage, but is not limited to them.
Hereinafter, the components of the medical member will be described in detail.

<Base material>

The base material of the present embodiment is a member that permeates light and has a polymer layer on an inner surface. In the present disclosure, the inner surface includes not only a surface inside the hollow base material, but also a surface of a member (impeller 14) in the hollow base material (casing 11) shown in FIG. 1A to FIG. 1B, a surface of a complexly uneven structure (through holes 23) shown in FIG. 2A and FIG. 2B, and an inner surface of an elongated tubular structure shown in FIG. 3A to FIG. 3B.
The base material may have an opening(s). The opening may be normally closed but have a mechanism or a structure for opening so as to, as described later, have a shape and structure capable of adsorbing a photopolymerization initiator or adsorbing a compound having both a photoreaction initiation group and a phosphorylcholine group. The shape, size, and number of the opening (s) are not particularly limited. For example, the base material may be reticulated

or porous and have a large number of small openings. Alternatively, as long as the inner surface of the base material can be exposed to light of a sufficient intensity, metal vapor deposition may be applied to a part or all of the outer surface of the base material, or a metal mesh or the like may be used in combination with the base material.

The base material is a member that allows light to pass through. The light may be, for example, ultraviolet rays having a wavelength of 10 to 400 nm. For example, ultraviolet rays having a wavelength of 200 nm to 380 nm can be appropriately used as the light for polymerization of MPC, a base material as described later. Specifically, the light permeability at least at a part of the base material can be set to 10% or more. When the light permeability at least at a part of the base material is 20% or more, for example, the irradiation time can be reduced. The light permeability varies depending on the type and thickness of the material constituting the base material. For example, when the material constituting the base material is a polyolefin member, the thickness can be set to 0.05 to 2.0 mm. Alternatively, when the base material is a polyolefin member and has a thickness of 0.1 to 1.0 mm, for example, the light permeability and durability are improved.

In addition to being able to permeate a sufficient amount of light for forming the polymer layer, the base material may have deterioration resistance to the incident light.

The base material is constituted by a light-permeating member that is, for example, a polyolefin member or an aromatic polyetherketone member.

Examples of the polyolefin member include polyethylene, low density polyethylene (LDPE), linear low density polyethylene (LLDPE), high density polyethylene (HDPE), ultra-high molecular weight polyethylene (UHMWPE), and the like. HDPE and UHMWPE have excellent chemical resistance and biocompatibility, and are suitable as materials of the medical member used in a living body.

Examples of the aromatic polyetherketone member include thin layer materials such as polyetherketone (PEK), polyetheretherketone (PEEK), polyetherketoneketone (PEKK), polyetheretherketoneketone (PEEKK), and polyetherketoneetherketoneketone (PEKEKK). PEEK has excellent chemical resistance and mechanical properties, and is preferably used as a medical member in a living body.

Such a base material can be obtained by putting powdery, granular, or pelletized polyolefin or aromatic polyetherketone into a mold and performing compression molding, extrusion molding, or injection molding. The base material obtained by the molding may be subjected to the polymer layer forming process as it is. A sheet-like or film-like base material rolled to be a hollow shape (cylindrical shape) may be heat-welded at a high frequency or the like, shaped by additional processes such as cutting, and subjected to a polymer layer forming process. The polyolefin and aromatic polyetherketone are thermoplastic resin, but have low fluidity even at a melting temperature or higher. Therefore, a solid polyolefin or aromatic polyetherketone can be put into a mold so as to be molded under high temperature and high pressure conditions.

The base material may be molded by adding a reinforcing material such as an antioxidant, a cross-linking agent, and carbon fiber. The base material is not limited to be formed of only one kind of resin, but may include multiple resins having different structures or physical properties, for example, that are kneaded and molded.

<Polymer Layer>

The polymer layer of the present embodiment contains a polymer having a phosphorylcholine group, for example, a polymer chain obtained by polymerizing a compound having a phosphorylcholine group, and the polymer is covalently bonded to the base material as a graft chain. Specifically, the polymer layer formed by bonding the phosphorylcholine group to the base material via the methacryloyloxyalkyl group is chemically stable and easily formed by polymerization.

The thickness of the polymer layer is preferably 5 to 200 nm, more preferably 10 to 100 nm. When the thickness is 5 nm or more, high antifouling property against blood, body fluid, and the like can be obtained. For example, when embedded in a living body and used, the polymer layer can effectively suppress protein adsorption. This makes it possible to obtain a medical member having excellent durability.

The polymer layer is provided at least at a part of the inner surface of the base material. The area where the polymer layer is provided includes an area (first area) where light capable of forming the polymer layer can reach through the base material when light of a predetermined intensity is emitted from the outer surface side of the base material.

For example, when the centrifugal blood pump 1 shown in FIG. 1A to FIG. 1B is irradiated with light of a predetermined intensity from the left side of FIG. 1B, light capable of forming a polymer layer passes through the base material 11 and reaches the inner surface of the base material 11 and the surface of the impeller 14.

The "light of a predetermined intensity" is light of an intensity that does not deteriorate the base material by irradiation with the light (the intensity depends on the material of the base material, and 10 $mW/cm^2$ or less in the case of polyethylene, for example). The "light capable of forming a polymer layer" also depends on the kinds of the base material and the polymer layer, and is light having an intensity of 1 $mW/cm^2$ or more in the case of polyethylene, for example.

The area where the polymer layer is formed may further include an area (second area) that is not directly irradiated with light capable of forming the polymer layer when irradiated with light of a predetermined intensity from the opening.

For example, in the base material 3 of FIG. 3A, the area 34A near the middle of the inner surface 34 is away from both the openings 32, 33. Therefore, when the base material 31 does not permeate light, the area 34A is irradiated with greatly attenuated light as compared with the light at the outer surface of the base material (near the opening 32 or 33). In particular, the light of a short wavelength such as ultraviolet rays is easily attenuated in the atmosphere or water, and it is difficult to directly irradiate the area 34A with light of sufficient intensity through the small opening 32 or 33. By increasing the light emitted from the light source, the light reaching the area 34A through the opening 32 or 33 can also be increased. However, very strong light entering the vicinity of the opening 32 or 33 in such cases would likely to deteriorate the base material in the vicinity of the opening 32 or 33.

<Method for Manufacturing Medical Member>

Next, the method for manufacturing the medical member of the present embodiment will be specifically described, including a forming method of the polymer layer.

First, a hollow base material that is permeable to light is formed. For example, as described above, polyolefin or aromatic polyetherketone is used as a material and molded to be a hollow shape by a known method.

Next, as described below, a polymer layer is formed on the surface of the base material by graft polymerization including covalent bonding. The graft polymerization is roughly classified into (1) a so-called "grafting from" method including surface-initiated graft polymerization and (2) a so-called "grafting to" method including graft-bonding of a polymer adsorbed on a base material (a polymer material for forming the polymer layer is adsorbed on the surface of the medical member by dip coating or the like, and bonded by cross-linking or using a reactive functional group).

An example of the graft polymerization method is a photo-initiated graft polymerization method in which a graft polymerization reaction is started in response to irradiation with light of a predetermined wavelength. A radical initiator can be mainly used as a polymerization initiator for the photo-initiated graft polymerization. As described later, in the graft polymerization method, a radical initiator is adsorbed on (applied to) a surface to be treated in advance in the above (1) "grafting from" method, and the polymer having a photoreaction initiation group is adsorbed on (applied to) a surface to be treated in advance in the above (2) "grafting to" method.

When a compound having a phosphorylcholine group (hereinafter, a PC compound) is grafted in a predetermined area on the surface of the base material (inner surface in the present embodiment) by the "grafting from" method, a photopolymerization initiator is usually adsorbed in the predetermined area. As a method of adsorbing the photopolymerization initiator, for example, the base material is immersed in an organic solvent to which the photopolymerization initiator is added, and after a predetermined time, is dried for removal of the organic solvent.

Next, the predetermined area is brought into contact with a solution of a polymerizable monomer which is the PC compound. Specifically, in the present embodiment, a base material that is permeable to light and molded to be a predetermined shape is immersed in a solution of the polymerizable monomer which is the PC compound and a water-soluble inorganic salt (hereinafter, "polymerization treatment liquid").

The polymerizable monomer used in this embodiment is, for example, 2-methacryloyloxyethylphosphorylcholine (hereinafter referred to as "MPC"), 2-acryloyloxyethylphosphorylcholine, 4-methacryloyloxybutylphosphorylcholine, 6-methacryloyloxyhexylphosphorylcholine, ω-Metachlorooxyethylenephosphorylcholine, 4-styryloxybutylphosphorylcholine, and the like.

The MPC has a chemical structure as shown in the following structural formula and is a polymerizable monomer having a phosphorylcholine group and a polymerizable methacrylic acid unit. The MPC is known to be easily polymerized by radical polymerization to form a high-molecular-weight homopolymer (Ishihara et al.: Polymer Journal, Vol. 22, p. 355 (1990)). Therefore, the PC compound in the polymerization treatment liquid of the present embodiment actually includes not only the polymerizable monomer but also a homopolymer.

[Chem1]

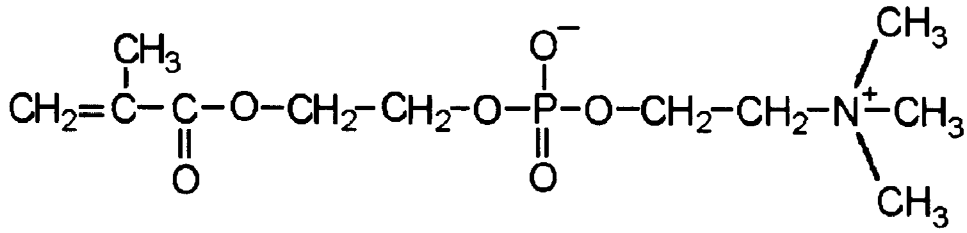

The water-soluble inorganic salt used in this embodiment is an alkali metal salt or an alkaline earth metal salt. One or more alkali metal salt is selected from a group including a sodium salt, potassium salt, lithium salt, and cesium salt. One or more alkaline earth metal salt is selected from a group including a calcium salt, strontium salt, barium salt, and radium salt.

Next, the base material is irradiated with light from the outer surface side. Specifically, while the polymerization treatment liquid is in contact with at least the inner surface of the base material that has undergone the above steps, the base material is irradiated with light (for example, ultraviolet rays) from the outer surface side.

In order that the PC compound is grafted at the predetermined area on the surface of the base material (inner surface in this embodiment) by the "grafting to" method, the PC compound (polymer) including both a photoreaction initiation group and a phosphorylcholine group are usually adsorbed in (applied to) the predetermined area. As a method of adsorbing the PC compound, for example, the base material is immersed in an organic solvent to which the PC compound is added for a predetermined time and then dried for removal of the organic solvent. Next, the base material is irradiated with light (for example, ultraviolet rays) from the outer surface side.

The polymer having both the photoreaction initiation group and the phosphorylcholine group, which is the PC compound used in the present embodiment, is, for example, a copolymer of 2-methacryloyloxyethyl phosphorylcholine, butylmethacrylate, and benzophenonemethacrylate (hereinafter, "PMBBP"), a copolymer of 2-acryloyloxyethyl phosphorylcholine, butylmethacrylate, and benzophenonemethacrylate, and a copolymer of 2-methacryloyloxyethylphosphorylcholine, butylmethacrylate, and acetophenone.

<Regarding Light Irradiation>

Depending on the kind of polymerization initiator/polymerization initiating group, light (for example, ultraviolet rays) of a suitable wavelength range is used for irradiation.

The ultraviolet irradiation light source of the present embodiment is, for example, a high-pressure mercury lamp (UVL-400HA manufactured by Riko Kagaku Sangyo Co., Ltd.), a light emitting diode lamp (MeV365-P601JMM manufactured by YEV Co., Ltd.), and a UV crosslinker (CL-1000 manufactured by Funakoshi Co., Ltd.), and the like.

The photopolymerization initiator of the present embodiment is, for example, an acetophenone-based photopolymerization initiator, a benzophenone-based photopolymerization initiator, an alkylphenone-based photopolymerization initiator, an acylphosphine oxide-based photopolymerization initiator, and an oxime ester-based photopolymerization agent. Preferably, acetophenone, benzophenone, benzyl, 1,4-dibenzoylbenzene, 2-isopropylthioxanthone, and 1-[4-(2-hydroxyethoxy)-phenyl]-2-hydroxy-2-methyl-1-propane-1-on.

Because the base material of the present embodiment has light permeability, without deterioration or embrittlement of the surface directly irradiated with light, the surface not directly irradiated with light is also irradiated with light of sufficient intensity. Therefore, it is possible to form a polymer layer even in an area that is difficult to be directly irradiated with light emitted by an irradiation light source outside the base material.

In this way, a polymer layer of a polymerized compound having a covalently bonded phosphorylcholine group is formed at least at a part of the inner surface of the base material.

In the present embodiment, the area (or surface) where the light emitted by the irradiation light source first reaches the base material is defined as an "exposed surface". The area (or surface) where the light entering the base material from the exposed surface passes through the base material and is emitted is defined as a "back exposed surface"

That is, in the above-mentioned "grafting from" method, in response to the light irradiation, photopolymerization initiator radicals are generated, and protons are extracted from the surface of the base material. As a result, a polymerization initiation point is formed on the surface of the base material, and the terminal of the PC compound reacts with the polymerization initiation point. As a result, the PC compound is covalently bonded to the surface of the base material as a graft chain, and the PC compound further grows into a graft polymer chain by a polymerization reaction. For example, because the entire hollow (cylindrical) base material 31 having a uniform thickness as in FIG. 3A permeates light, not

only on the exposed surface but also on the back exposed surface of the base material 31 are irradiated with light of sufficient intensity. As a result, the photo-initiated graft polymerization occurs and a polymer layer is formed on or in the base material 31 in an entire area where the photopolymerization initiator is adsorbed and that is in contact with the polymerization treatment liquid. When the organic solvent or the polymerization treatment liquid to which the photopolymerization initiator is added is filled inside the base material 31, the polymer layer is formed only on the inner surface 34 of the base material 31.

Alternatively, in the "grafting to" method, in response to the light irradiation, radicals are generated from the photoreaction initiation group of the PC compound, having a structure including both the photoreaction initiation group and the phosphorylcholine group, such that the polymerization initiation point is formed in the PC compound and reacts with the functional groups on the surface of the base material. As a result, the PC compound is covalently bonded to the surface of the base material and forms a graft polymer layer. Because the entire hollow (cylindrical) base material 31 having a uniform thickness as in FIG. 3A permeates light, not only the exposed surface but also the back exposed surface of the base material 31 are irradiated with light of sufficient intensity. As a result, the photo-initiated graft bonding occurs and a polymer layer is formed on or in the base material 31 in the entire area where the compound having both the photoreaction initiation group and the phosphorylcholine group is adsorbed. When the organic solvent to which the compound having both the photoreaction initiation group and the phosphorylcholine group is added is filled inside the base material 31, the polymer layer is formed only on the inner surface 34 side of the base material 31.

As described above, using either the "grafting from" method or the "grafting to" method, the polymer chain of the PC compound can be covalently bonded and stably fixed to the surface of the base material by the photo-initiated graft polymerization.

The above-described polymer layer of the present embodiment includes a homopolymer composed of a single kind of polymerizable monomer having a phosphorylcholine group, but the polymer layer is not limited to this. The polymer layer may include a copolymer composed of a polymerizable monomer having a phosphorylcholine group and another (meth)acrylate compound monomer (vinyl compound), for example. Depending on the kind of the other vinyl compound, the polymer layer can have a function such as mechanical strength improvement.

The medical member of the present embodiment can be manufactured through the above steps.

According to the medical member of the presently disclosed embodiment manufactured in this manner, by the MPC polymerization treatment under predetermined conditions (MPC (polymer) concentration, light irradiation intensity, and light irradiation time), it is possible to form the MPC polymer layer not only on the exposed surface that is directly irradiated with light but also on the back exposed surface. Because the MPC polymer layer can be thus formed on the back exposed surface, it is possible to form the MPC polymer layer through the photo-initiated graft polymerization by the "grafting from" method or photo-initiated graft bonding by the "grafting to" method even on an inner surface of an elongated tubular member, an inner surface of a complicated surface structure (concavo-convex structure, etc.), and inside of pores of a porous member, including the area which is difficult to be directly irradiated with light of sufficient intensity.

As a result, it is possible to exhibit high biocompatibility and high antifouling property even on such a surface. That is, in the living body, in addition to biocompatibility (bioaffinity) such as blood compatibility (affinity) and hydrophilicity, the antifouling property can be exerted for a relatively long period of time such that adsorption of protein and cell adhesion on the surface of the substrate are suppressed. As a result, it is possible to effectively reduce partly blocking of the hollow structure or filling of a fine uneven structure or pores due to the protein adsorbed on the surface of the base material (antithrombotic property and the like), and the necessary functions can be exhibited for a long period of time as compared with the case where a polymer layer is formed by physical adsorption, for example.

For example, the presently disclosed embodiment can be specifically applied to an artificial blood vessel, a hollow thread, an artificial heart pump, a stent (for vasodilation), and a dialyzer and exert at least one of antithrombotic and blood compatible effects. Furthermore, the presently disclosed embodiment can be applied to a skull plate, a catheter, a stent (other than for vasodilation), a nerve induction tube, a shunt tube, a spinal cage, and an artificial lung and effectively exert antifouling property.

When the PC compound is grafted on the surface of a base material containing a photopolymerization initiating group in its molecular structure (for example, PEEK) as a modification, the step of adsorbing the photopolymerization initiator is not necessary.

[EXAMPLE 1]

<Examination of Ultraviolet Permeability with respect to Thickness of Base Material>

Four polyethylene sheets (density: 0.93 g/$cm^3$) having different thicknesses were prepared as the base materials. The size of the base materials was 50 mm in length and 10 mm in width, and the respective thicknesses of the base materials were 0.4 mm, 0.6 mm, 0.8 mm, and 1.0 mm.

Each of the prepared base materials was irradiated with ultraviolet rays such that the angle of incidence on

one surface (exposed surface) was 0 degree. As the ultraviolet intensity $UV_a$ at the exposed surface was changed within the range of 1 to 15 mW/cm$^2$, the ultraviolet intensity $UV_b$ at the other surface (back exposed surface) was measured, and the ultraviolet permeability $UV_r$ (%) was calculated by the following formula (1).

$$UV_r = \{UV_a/UV_b\}x100 \qquad \text{Formula (1)}$$

FIG. 4 shows the ultraviolet permeability when the thickness of the base material was changed. The ultraviolet permeability $UV_r$ calculated by the Formula (1) changed depending on the thickness of the base material, about 67% when the thickness was 0.4 mm, about 63% when the thickness was 0.6 mm, about 54% when the thickness was 0.8 mm, and about 36% when the thickness was 1.0 mm.

[EXAMPLE 2]

<Examination of UV Permeability with respect to Density of Base Material>

Four polyethylene sheets (density: 0.92 g/cm$^3$, 0.925 g/cm$^3$, 0.935 g/cm$^3$, and 0.96 g/cm$^3$) having different densities were prepared as the base materials. The size of each of the base materials was 50 mm in length, and 10 mm in width, and 0.6 mm in thickness.

Each of the prepared base materials was irradiated with ultraviolet rays such that the angle of incidence on the exposed surface was 0 degree. As the ultraviolet intensity $UV_a$ at the exposed surface was changed within the range of 1 to 15 mW/cm$^2$, the ultraviolet intensity $UV_b$ at the back exposed surface was measured, and the ultraviolet permeability $UV_r$ was calculated by the above Formula (1).

FIG. 5 shows the ultraviolet permeability when the density of the base material was changed. The ultraviolet permeability $UV_r$ calculated by the Formula (1) changed depending on the density of the base material, about 66% when the density was 0.92 g/cm$^3$ and about 45% when the density was 0.96 g/cm$^3$.

[EXAMPLE 3]

<MPC Polymer Layer on Exposed Surface and Back Exposed Surface>

Among the base materials used in Example 1, one having a thickness of 1.0 mm was subjected to MPC polymerization treatment of the exposed surface and the back exposed surface by the "grafting from" method.

The base material was immersed in an acetone solution of benzophenone (1.0 g/dL) as a polymerization initiator for 30 seconds and then immediately pulled up, and the solvent was removed at room temperature so that the benzophenone was adsorbed on both surfaces of the base material.

Next, while the base material sufficiently degassed and sufficiently adsorbed with benzophenone was immersed in a polymerization treatment liquid including MPC monomer at 0.3 mol/L and sodium chloride at 2.5 mol/L kept at the temperature of 60°C in advance, the base material was irradiated with ultraviolet rays (wavelength: 300 to 400 nm) for 90 minutes.

After being pulled up from the polymerization treatment liquid, the base material was thoroughly washed with pure water and ethanol, and a base material treated with MPC polymer was obtained.

The exposed surface was irradiated with ultraviolet rays at the angle of incidence of 0 degrees and at the intensity of 5.0 mW/cm$^2$. Here, the ultraviolet permeability of the base material used in Example 1 having a thickness of 1.0 mm was 36%. Therefore, the ultraviolet intensity on the back exposed surface was 1.8 mW/cm$^2$.

The surface structure of the base material subjected to the MPC polymerization treatment as described above was analyzed by the following methods.

(1) Fourier Transform Infrared Spectroscopy

FIG. 6 shows results of Fourier Transform Infrared Spectroscopy (FT-IR) measurement by Attenuated Total Reflectance (ATR method) on each of the exposed surface and the back exposed surface of the base material subjected to the MPC polymerization treatment. The FT-IR measurement was performed using an FT-IR device (FT/IR-6300 type A manufactured by JASCO Corporation) with a resolution of 4 cm$^{-1}$ and an integration number of 64 times.

As shown in FIG. 5, the spectra obtained by the FT-IR measurement at the exposed surface and the back exposed surface had almost the same shape, each having peaks presumed to be derived from the MPC.

(2) TEM Image Observation

FIG. 7A and FIG. 7B show TEM images of cross sections of the base materials subjected to the MPC polymerization treatment. MPC polymer (PMPC) layers were formed on the exposed surface (FIG. 7A) and the back exposed surface (FIG. 7B), and the thickness of the formed layers were substantially the same.
As described above, it was confirmed that, under the conditions in Example 3, an MPC polymer layer having the same thickness (about 80 nm) was formed on the exposed surface and the back exposed surface of the base material of a light-permeating member having an ultraviolet permeability of 36%. By changing the conditions, MPC polymer layers having different thicknesses can be formed on the exposed surface and the back exposed surface.

[EXAMPLE 4]

<Change in Layer Thickness of MPC Polymer Layer depending on UV Intensity and UV Irradiation Time>

Among the base materials used in Example 1, one having a thickness of 0.6 mm (ultraviolet permeability: about 63%) was subjected to the MPC polymerization treatment by the "grafting from" method.
The MPC polymerization treatment was carried out under the same conditions as in Example 3 except that the ultraviolet intensity at the exposed surface was 2.5 $mW/cm^2$, 5.0 $mW/cm^2$, and 10.0 $mW/cm^2$.
Furthermore, the layer thickness of the MPC polymer layer formed on the back exposed surface of the base material was measured at the ultraviolet irradiation times of 15 minutes, 30 minutes, 60 minutes, and 90 minutes. The layer thickness was measured with a spectroscopic ellipsometer (Lincoln type manufactured by J.A. Woollam Co., Inc.). The measurement was performed at an angle of incidence at 70 degrees, and the layer thickness was calculated using a Cauchy model with a refractive index of 1.49 at a wavelength of 632.8 nm.
As described above and shown in FIG. 4, because the thickness of the base material was 0.6 mm (ultraviolet permeability: about 63%), when the ultraviolet intensity on the exposed surface was 5.0 $mW/cm^2$, for example, the ultraviolet intensity on the back exposed surface was about 3.2 $mW/cm^2$. As a result of Example 4, when the ultraviolet intensity on the exposed surface was 5.0 $mW/cm^2$, the formation of the MPC polymer layer was not clearly confirmed at the ultraviolet irradiation time of 30 minutes or less, as shown in FIG. 8. At the ultraviolet irradiation time of 60 minutes, the formation of an MPC polymer layer having a thickness of about 80 nm was confirmed. Furthermore, at the ultraviolet irradiation time of 90 minutes, there was no change in the thickness, and it was about 80 nm.
Specifically, the following results were obtained depending on the ultraviolet intensity on the exposed surface. When the ultraviolet intensity on the exposed surface was 10.0 $mW/cm^2$ (the ultraviolet intensity on the back exposed surface was about 6.3 $mW/cm^2$), an MPC polymer layer of about 30 nm was formed by irradiation for 15 minutes, and about 80 nm by irradiation for 30 minutes or more. There was no change in the layer thickness after 30 minutes. When the ultraviolet intensity on the exposed surface was 5.0 $mW/cm^2$ (the ultraviolet intensity on the back exposed surface was about 3.2 $mW/cm^2$), the thickness of the formed MPC polymer layer was about 5 nm or less by ultraviolet irradiation for 15 minutes and 30 minutes, but it was about 80 nm by ultraviolet irradiation for 60 minutes or more. When the ultraviolet intensity on the exposed surface was 2.5 $mW/cm^2$ (the ultraviolet intensity on the back exposed surface was about 1.6 $mW/cm^2$), the thickness of the formed MPC polymer layer was about 5 nm or less by ultraviolet irradiation for 15 minutes and about 15 nm by ultraviolet irradiation for 30 minutes. The thickness did not change by ultraviolet irradiation for 90 minutes or more, and was about 60 nm.
According to Example 4, the thickness of the MPC polymer layer to be formed is determined by the irradiation conditions on the surface on which the MPC polymer layer is formed, regardless of whether it is the exposed surface or the back exposed surface. Therefore, for example, in order to form an MPC polymer layer having a thickness of 5 nm or more within an ultraviolet irradiation time of 15 minutes, it was necessary to set the ultraviolet intensity on the back exposed surface at about 2.5 $mW/cm^2$ or more. Furthermore, by increasing the ultraviolet intensity on the exposed surface, the ultraviolet intensity on the back exposed surface can also be increased, and the MPC polymer layer having a desired thickness can be formed in a short time. Further, from the viewpoint of not deteriorating the base material, the ultraviolet intensity on the exposed surface can be set to be about 10 $mW/cm^2$. Furthermore, the thickness of the base material capable of achieving the ultraviolet intensity of about 2.5 $mW/cm^2$ or more (permeability of 25%) at the back exposed surface when the ultraviolet intensity at the exposed surface was 10 $mW/cm^2$ was 1.3 mm or less, assuming an approximate curve (linear) of FIG. 4. An approximate straight line (not shown) of the data in FIG. 4 can be represented by a formula $y = -49.936x + 89.835$ (in the formula, x is the PE Thickness and y is UV Permeability).

[EXAMPLE 5]

<Change in Layer Thickness of MPC Polymer Layer formed on Back Exposed Surface depending on MPC Monomer Concentration>

Among the base materials used in Example 1, one having a thickness of 0.6 mm (ultraviolet permeability: about 63%) was subjected to the MPC polymerization treatment by the "grafting from" method.

The MPC polymerization treatment was carried out in the same manner as in Example 3 except that the MPC monomer concentrations at the exposed surface (and the back exposed surface) were 0.10, 0.20, 0.225, 0.25, and 0.30 mol/L.

The layer thickness of the MPC polymer layer formed on the back exposed surface of the base material was measured at the ultraviolet irradiation time of 90 minutes in the same manner as in Example 4.

As a result, as shown in FIG. 9, an MPC polymer layer of 5 nm or more was formed at all MPC monomer concentrations of 0.10 mol/L or more. By further considering from a different viewpoint, it was confirmed that an MPC monomer concentration of 0.10 mol/L or more was enough to form an MPC polymer layer having a thickness of 5 nm or more at the ultraviolet irradiation time of 90 minutes or less.

[EXAMPLE 6]

<Change in Layer Thickness of MPC Polymer Layer formed on Back Exposed Surface depending on MPC Polymer Concentration>

Among the base materials used in Example 1, one having a thickness of 0.6 mm (ultraviolet permeability: about 63%) was subjected to the MPC polymerization treatment by the "grafting to" method. PMBBP was used as the MPC polymer.

The base material was immersed in an ethanol solution of MPC polymer at 0.05, 0.10, 0.15 or 0.20 wt% for 30 seconds and then immediately pulled up, and the solvent was removed at room temperature. After the solvent was completely removed, the base material was immersed in the MPC polymer solution again for 30 seconds and then immediately pulled up, and the solvent was removed at room temperature.

Next, in an irradiation device (UV cross linker (CL-1000 manufactured by Funakoshi Co., Ltd.)), the base material on which the MPC polymer was adsorbed was irradiated with ultraviolet rays (wavelength: 200 to 300 nm, intensity on the exposed surface: 5.0 mW/cm$^2$) for 10 minutes. After being taken out from the irradiation device, the base material was thoroughly washed with ethanol, and a base material subjected to with MPC polymerization treatment was obtained.

Because the thickness of the base material was 0.6 mm (ultraviolet permeability: about 63%), when the ultraviolet intensity at the exposed surface was 5.0 mW/cm$^2$ as described above, the ultraviolet intensity at the back exposed surface was about 3.0 mW/cm$^2$ according to FIG. 4.

The layer thickness of the MPC polymer layer formed on the back exposed surface of the base material was measured at each of the MPC polymer concentrations of 0.01, 0.02, 0.05, 0.10, 0.15, and 0.20 wt%. The layer thickness was measured by the same method as in the above Example 4.

As a result of Example 6, as shown in FIG. 10, an MPC polymer layer of 5 nm or more was formed at all MPC polymer concentrations of 0.01 wt% or more. By further considering from a different viewpoint, it was confirmed that an MPC polymer concentration of 0.01 wt% or more was necessary to form an MPC polymer layer having a thickness of 5 nm or more at the ultraviolet irradiation time of 10 minutes.

According to the above embodiments, an MPC polymer layer can be formed not only on the surface that is directly irradiated with light but also on the back exposed surface of a hollow base material that permeates light by the MPC polymerization treatment under predetermined conditions (MPC monomer or polymer concentration, light irradiation intensity, and light irradiation time). As a result, the MPC polymer layer can be formed by the photo-initiated graft polymerization also on the inner surface of the base material (including a surface of the internal structure of a hollow base material, an inner surface of an elongated tubular member, and a surface of a complicated uneven structure), which has been difficult until now. This improves the performance of the inner surface.

The present disclosure is not limited to the above-described embodiments, and various modifications may be made without departing from the gist of the present disclosure.

For example, the shape of the medical member according to the presently disclosed embodiment is not limited to those shown in FIG. 1A to FIG. 3B. It may be, for example, a tubular member with only one end open, a hollow spherical or box-shaped member with one or more openings, a porous member, and the like.

Furthermore, the polymer layer is formed on the entire inner surface of the base material in the above embodiment, but may be formed also on the outer surface of the base material. Furthermore, the polymer layer may be formed

only in the vicinity of the opening on the inner surface or only in the vicinity of the center (for example, the area 14A near the center in FIG. 3).

Industrial Applicability

The present disclosure can be applied to a medical member and a method for manufacturing the same.

## Claims

1. A medical member comprising:

   a base material that is permeable to light; and
   a polymer layer that has a phosphorylcholine group, the polymer layer located at least at a part of an inner surface of the base material,
   wherein the part of the inner surface includes a first area that is irradiated with light of an intensity capable of forming the polymer layer in response to being irradiated with light of a predetermined intensity from an outer surface side of the base material.

2. The medical member according to claim 1,
   wherein the base material has an opening, and
   wherein the part of the inner surface includes a second area that is not directly irradiated with light of the intensity capable of forming the polymer layer in response to being irradiated with light of the predetermined intensity through the opening.

3. The medical member according to claim 1 or 2, wherein the base material has a light permeability of 10 to 90%.

4. The medical member according to any one of claims 1 to 3, wherein the base material has a tubular shape.

5. The medical member according to any one of claims 1 to 4, wherein the base material is porous.

6. The medical member according to any one of claims 1 to 5, wherein the polymer layer having the phosphorylcholine group includes a 2-methacryloyloxyethylphosphorylcholine polymer.

7. A medical member comprising:

   a base material that is permeable to light; and
   a polymer layer that has a phosphorylcholine group, the polymer layer located at least at a part of an inner surface of the base material,
   wherein the inner surface of the base material is irradiated with light of a light intensity of 3 $mW/cm^2$ or less when light of a light intensity of 15 $mW/cm^2$ or less enters from an outer surface side of the base material.

8. A method for manufacturing a medical member, the method comprising:

   bringing an inner surface of a base material that is permeable to light into contact with a solution of a compound having a phosphorylcholine group;
   irradiating the base material with light from an outer surface of the base material; and
   forming a polymer layer having a phosphorylcholine group on at least at a part of the inner surface of the base material.

9. The method for manufacturing a medical member according to claim 8, further comprising:
   adsorbing a polymerization initiator on the inner surface of the base material.

10. The method for manufacturing a medical member according to claim 8 or 9, wherein, in the irradiating, the light has a light intensity of 15 $mW/cm^2$ or less at the outer surface of the base material, and the light has a light intensity of 3 $mW/cm^2$ or less at the inner surface of the base material.

11. The method for manufacturing a medical member according to any one of claims 8 to 10, wherein, in the irradiating,

the inner surface of the base material is in contact with the solution of the compound having a phosphorylcholine group.

**12.** The method for manufacturing a medical member according to claim 11, wherein, in the irradiating, the solution of the compound having a phosphorylcholine group includes a monomer having a phosphorylcholine group at a concentration of 0.10 mol/L or more and 2.00 mol/L or less.

**13.** The method for manufacturing a medical member according to claim 11 or 12, wherein, in the irradiating, the solution of the compound having a phosphorylcholine group further includes an inorganic salt.

**14.** A method for manufacturing a medical member, the method comprising:

adsorbing a compound that has a photoreaction initiation group and a phosphorylcholine group on a surface of a base material that is permeable to light by bringing the surface of the base material into contact with a solution of the compound;
irradiating the base material with light having a light intensity that is enough to excite the photoreaction initiation group of the adsorbed compound; and
forming a polymer layer by covalently bonding the compound at least at a part of the surface of the base material, wherein the polymer layer is formed on a back side of the surface of the base material that is directly irradiated with the light.

FIG.1A

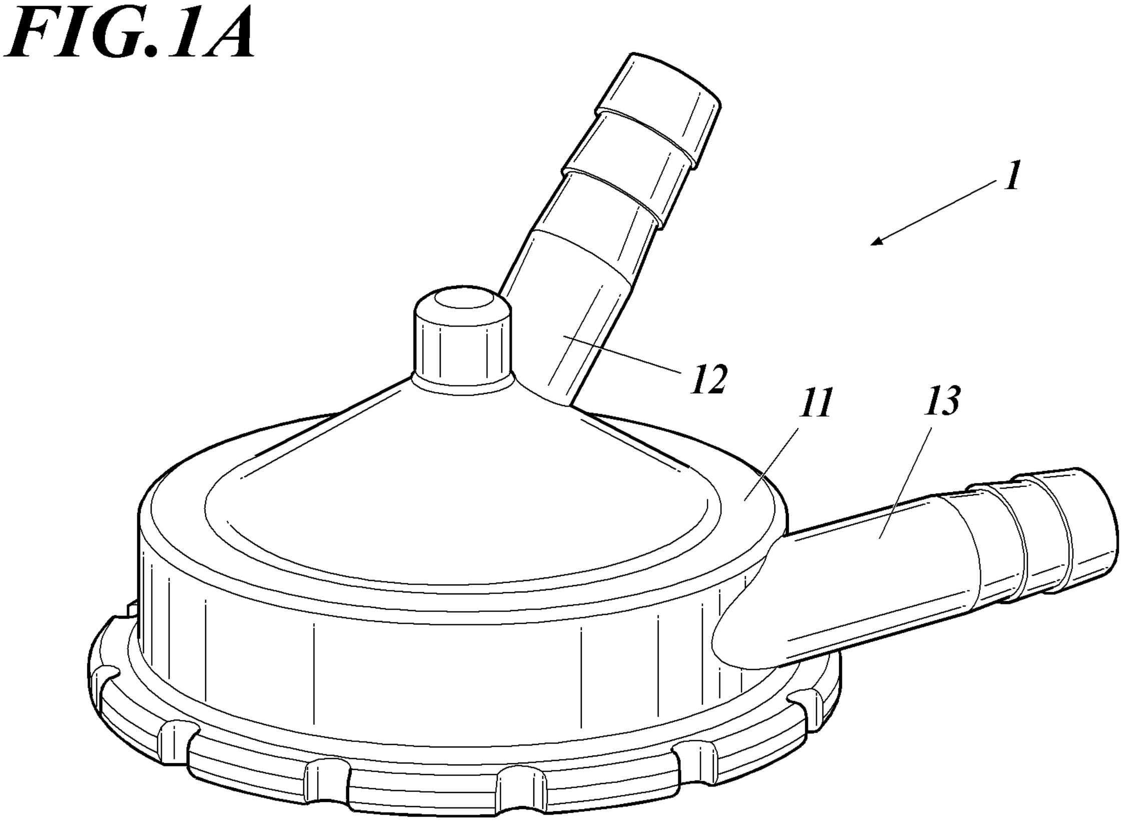


FIG.1B

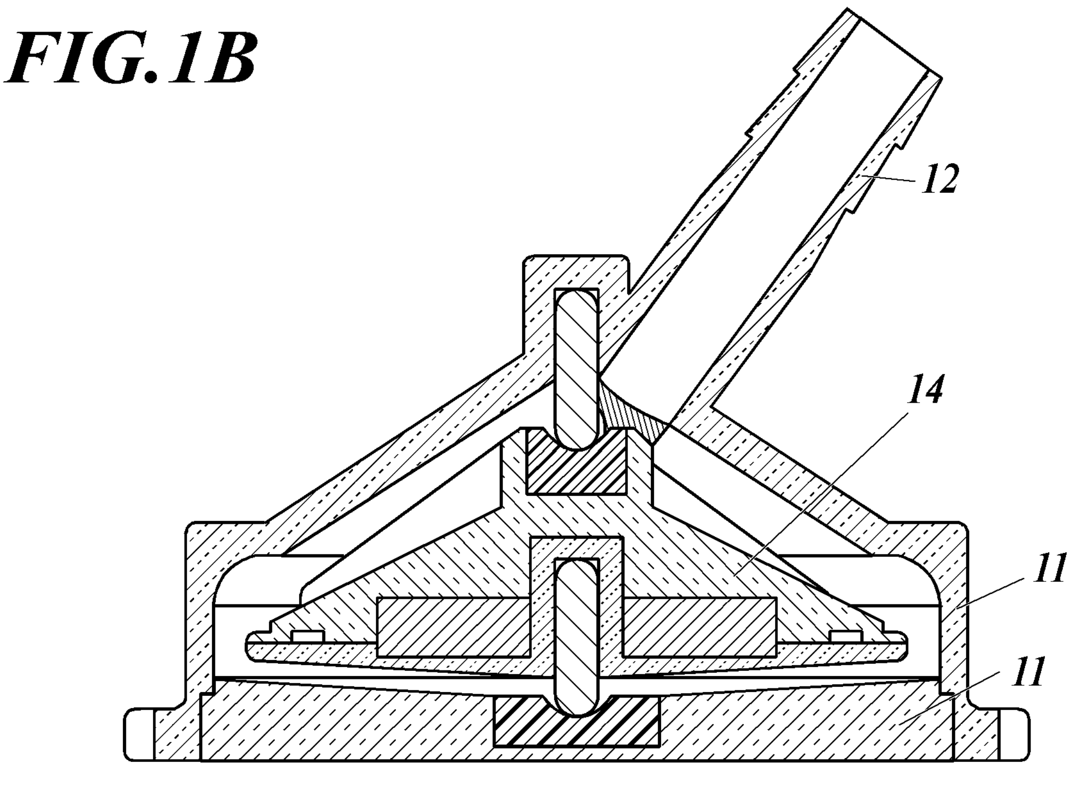

FIG.2A

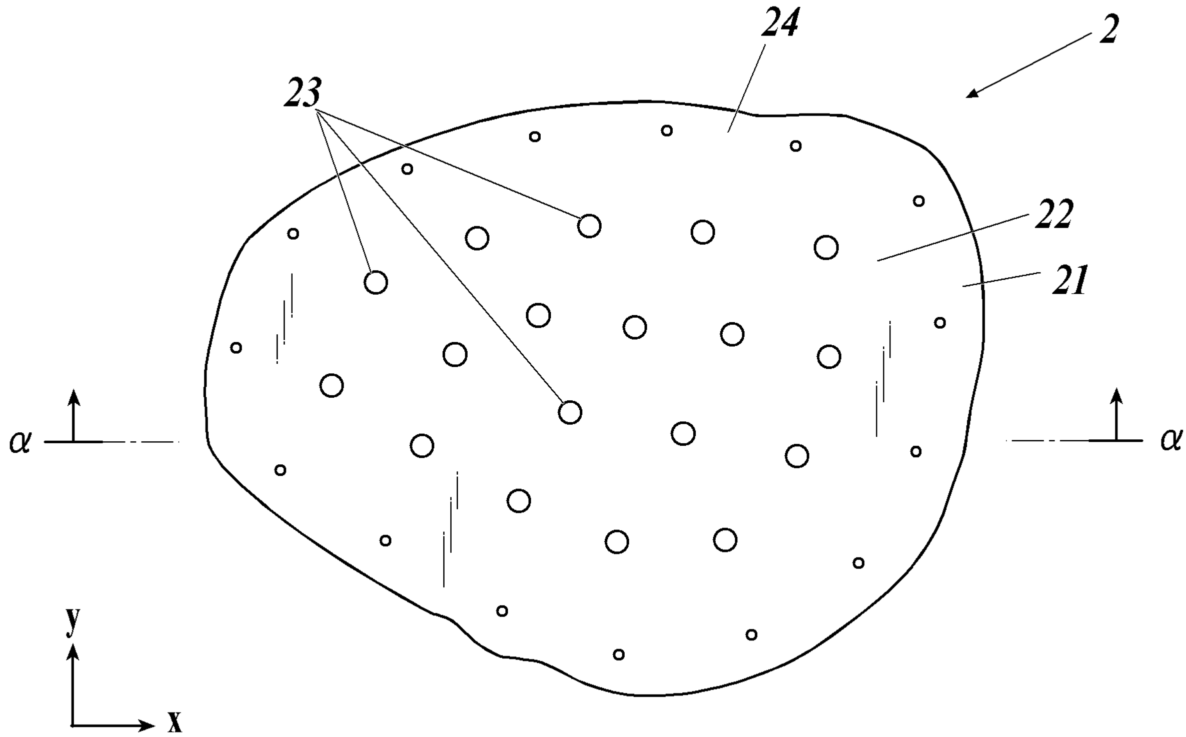


FIG.2B

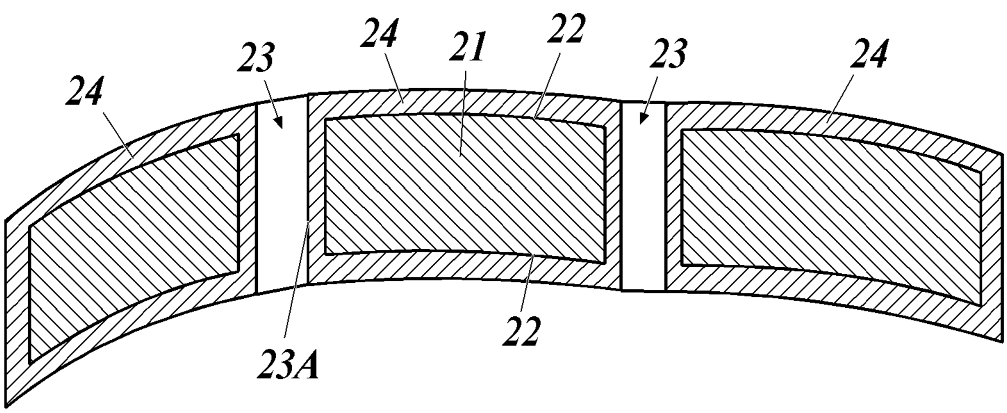

# FIG.3A

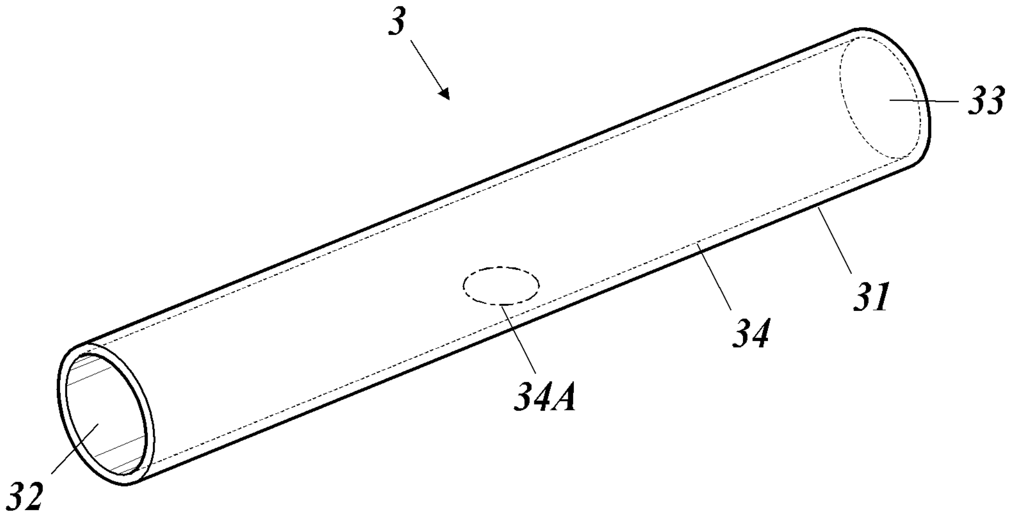


# FIG.3B

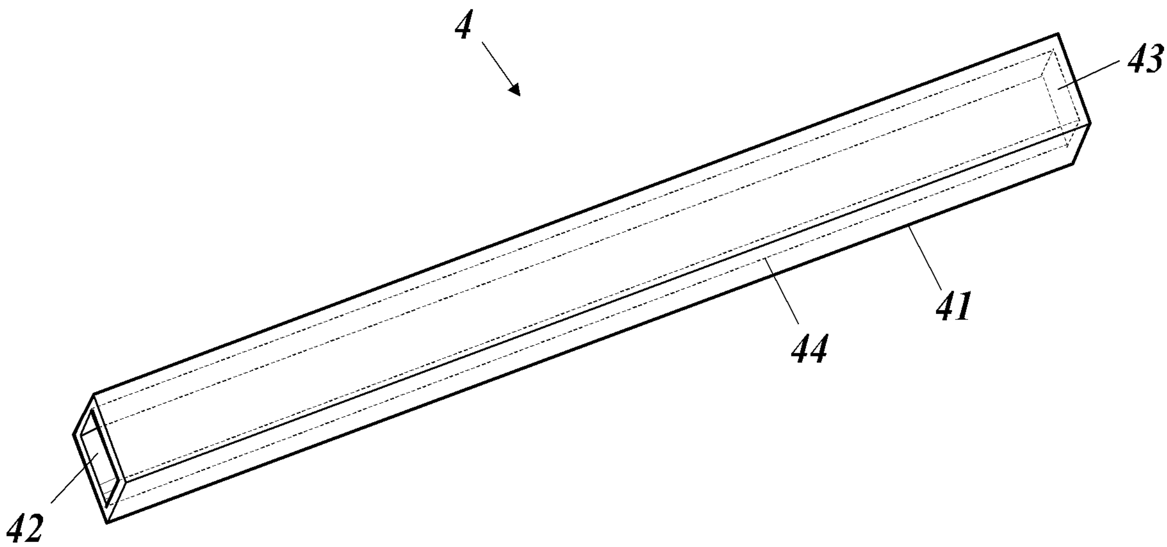

# FIG.4

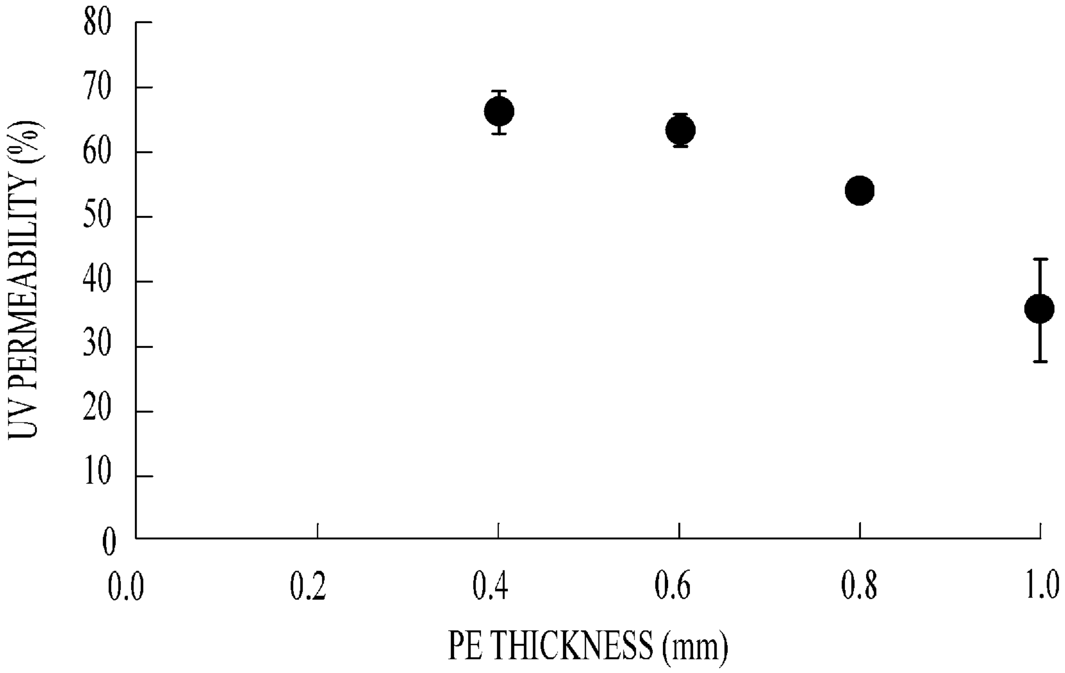
UV PERMEABILITY (%)
80
70
60
50
40
30
20
10
0
0.0
0.2
0.4
0.6
0.8
1.0
PE THICKNESS (mm)

# FIG.5

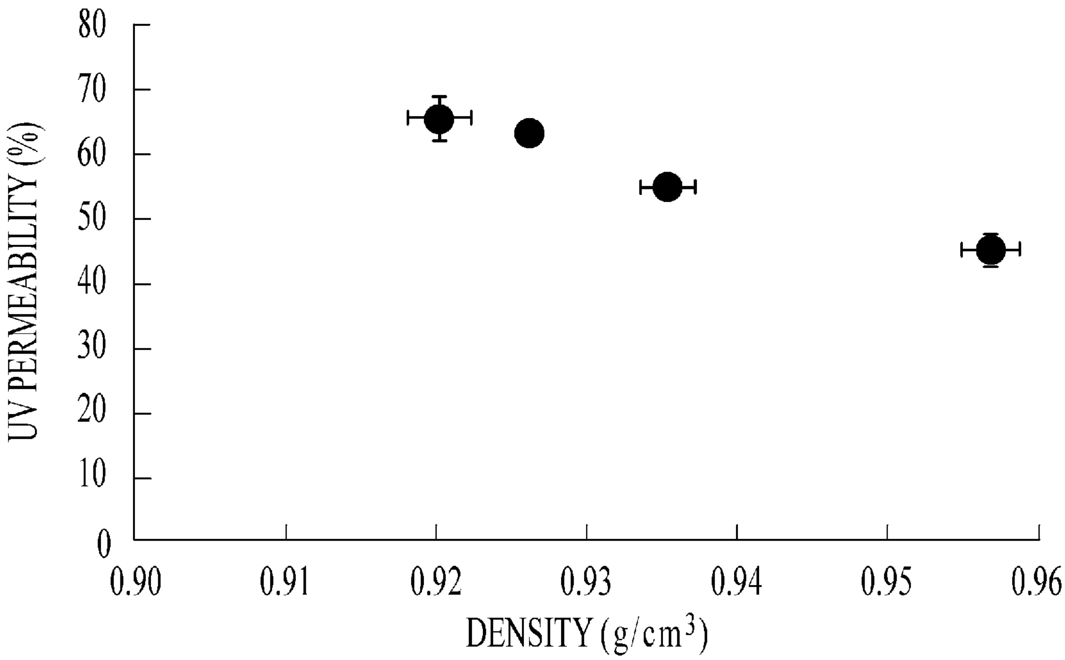
UV PERMEABILITY (%)
80
70
60
50
40
30
20
10
0
0.90
0.91
0.92
0.93
0.94
0.95
0.96
DENSITY (g/cm3)

*FIG.6*

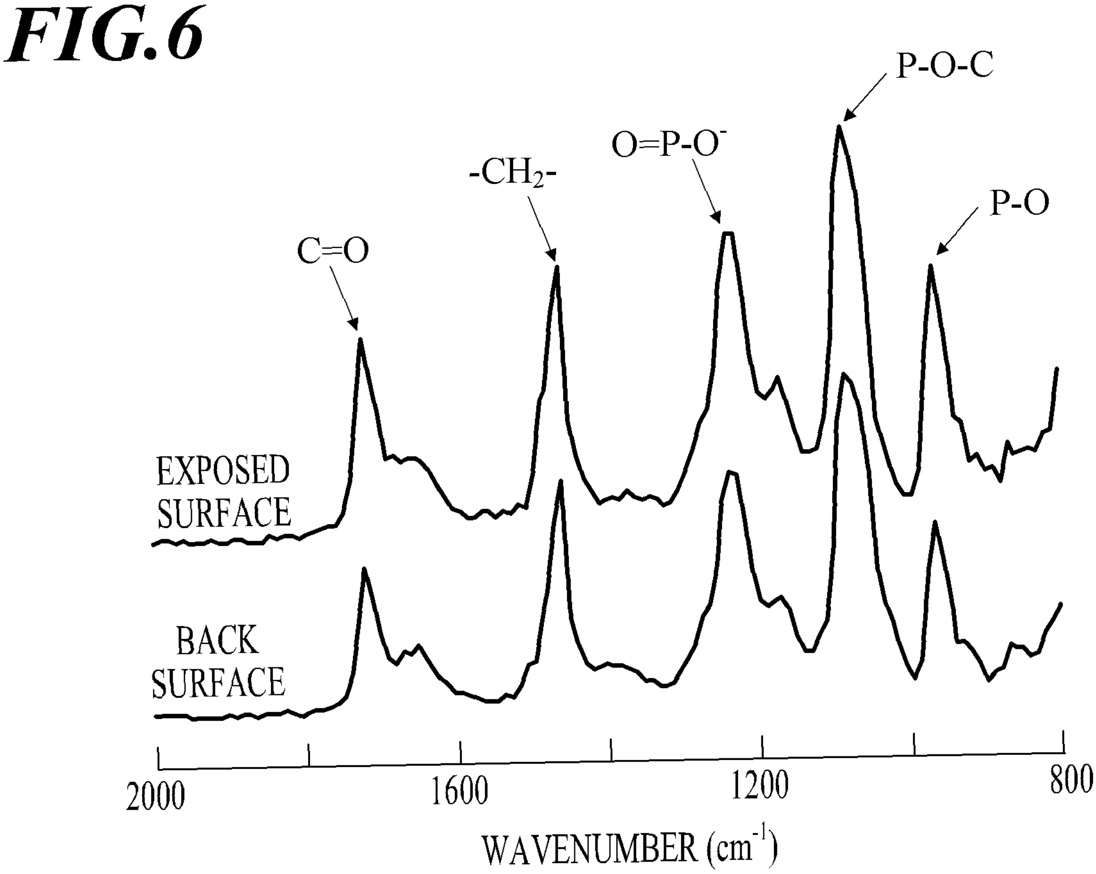
P-O-C
O=P-O⁻
-CH₂-
P-O
C=O
EXPOSED SURFACE
BACK SURFACE
2000
1600
1200
800
WAVENUMBER (cm⁻¹)

# FIG. 7A

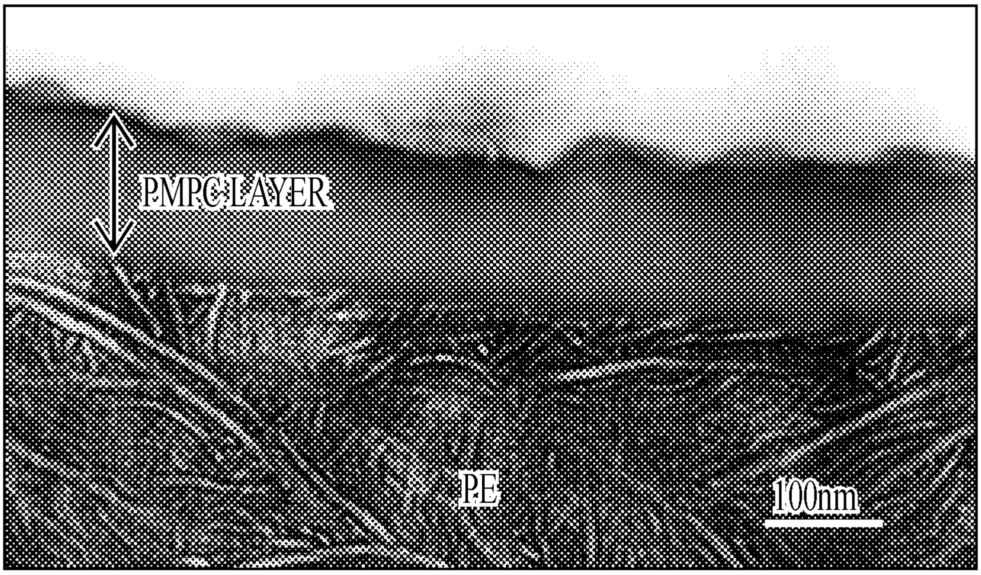


# FIG. 7B

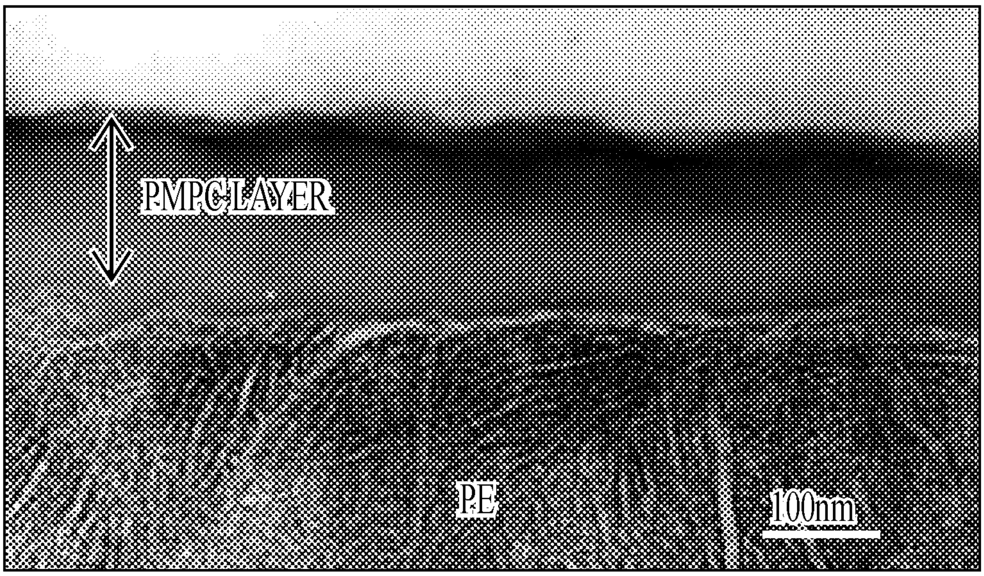

# FIG.8

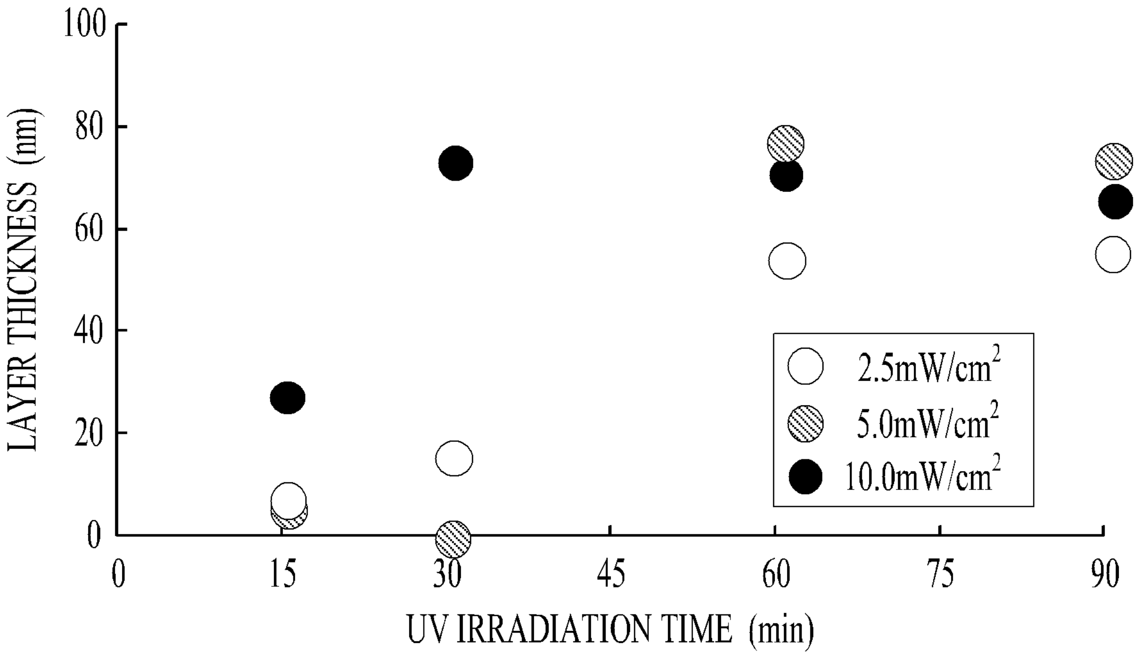
100
80
60
40
20
0
LAYER THICKNESS (nm)
0
15
30
45
60
75
90
UV IRRADIATION TIME (min)
2.5mW/cm2
5.0mW/cm2
10.0mW/cm2

# FIG.9

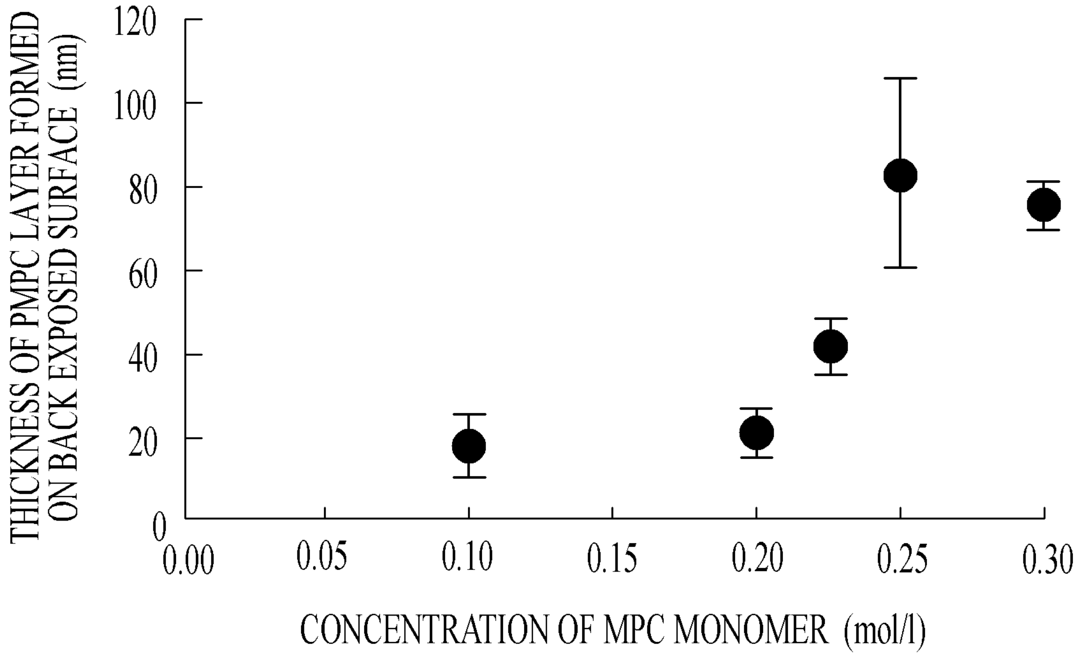
THICKNESS OF PMPC LAYER FORMED ON BACK EXPOSED SURFACE (nm)
120
100
80
60
40
20
0
0.00
0.05
0.10
0.15
0.20
0.25
0.30
CONCENTRATION OF MPC MONOMER (mol/l)

# FIG.10

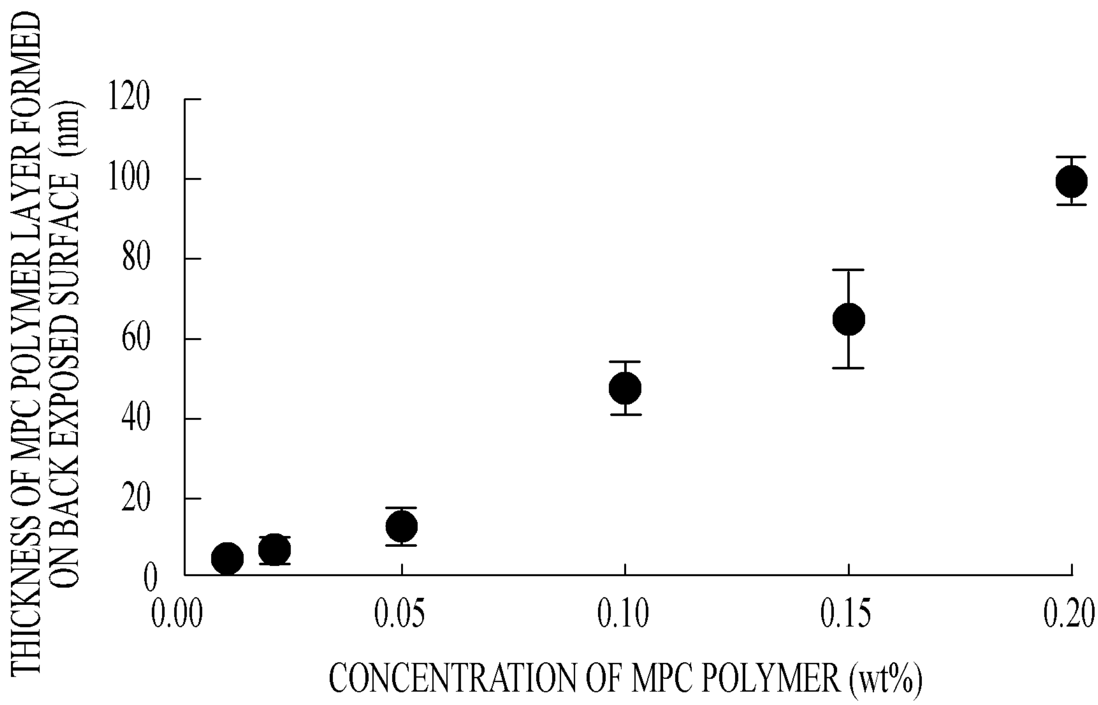
THICKNESS OF MPC POLYMER LAYER FORMED ON BACK EXPOSED SURFACE (nm)
120
100
80
60
40
20
0
0.00
0.05
0.10
0.15
0.20
CONCENTRATION OF MPC POLYMER (wt%)

**INTERNATIONAL SEARCH REPORT**

International application No. PCT/JP2019/036226

A. CLASSIFICATION OF SUBJECT MATTER
Int.Cl. See extra sheet

According to International Patent Classification (IPC) or to both national classification and IPC

B. FIELDS SEARCHED

Minimum documentation searched (classification system followed by classification symbols)
Int.Cl. A61L27/44, A61L27/16, A61L27/18, A61L27/50, A61L27/56, A61L29/04, A61L29/06, A61L29/08, A61L29/12, A61L29/14, A61L31/04, A61L31/06, A61L31/10, A61L31/12, A61L31/14

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2015-500088 A (ABBOTT CARDIOVASCULAR SYSTEMS INC.) 05 January 2015, claim 1, paragraphs [0005], [0007], [0008], [0018] & US 2012/0077049 A1, claim 1, paragraphs [0008], [0009], [0025] & WO 2013/085562 A1 & EP 2680893 A1 & CN 103533967 A & KR 10-2014-0101285 A | 1-14 |
| Y | WO 2009/081870 A1 (JAPAN MEDICAL MATERIALS CORPORATION) 02 July 2009, claims 1, 3, 8, 10, paragraph [0037] & US 2011/0027757 A1, claims 1, 3, 8, 10, paragraph [0067] & EP 2233159 A1 | 1-14 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 November 2019 (18.11.2019) | 03 December 2019 (03.12.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/036226

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2015-073786 A (KYOCERA MEDICAL CORPORATION) 20 April 2015, claim 1, paragraphs [0013], [0021], [0047], [0056], fig. 1-4 & US 2016/0213476 A1, claim 1, paragraphs [0032], [0045], [0087], [0098]-[0100], fig. 1-4 & WO 2015/053203 A1 & EP 3056226 A1 & CN 105611951 A | 1-14 |
| Y | JP 2017-144191 A (KYOCERA CORP.) 24 August 2017, claim 1, paragraph [0008] & WO 2017/142047 A1 | 1-14 |
| A | JP 03-103264 A (KANEGAFUCHI CHEM IND CO., LTD.) 30 April 1991, page 5, lower right column, lines 4-8 & US 5128170 A column 9, lines 10-17 & EP 397130 A2 & DE 69018691 C | 1-14 |

Form PCT/ISA/210 (continuation of second sheet) (January 2015)

**INTERNATIONAL SEARCH REPORT**

International application No.
PCT/JP2019/036226

CLASSIFICATION OF SUBJECT MATTER
A61L27/44(2006.01)i, A61L27/16(2006.01)i, A61L27/18(2006.01)i, A61L27/50(2006.01)i, A61L27/56(2006.01)i, A61L29/04(2006.01)i, A61L29/06(2006.01)i, A61L29/08(2006.01)i, A61L29/12(2006.01)i, A61L29/14(2006.01)i, A61L31/04(2006.01)i, A61L31/06(2006.01)i, A61L31/10(2006.01)i, A61L31/12(2006.01)i, A61L31/14(2006.01)i

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ISHIHARA et al.** *Polymer Journal,* 1990, vol. 22, 355